# EUROPÄISCHE PATENTSCHRIFT

(11) **EP 0 999 820 B1**
(45) Veröffentlichungstag und Bekanntmachung des Hinweises auf die Patenterteilung: **25.09.2002**
(21) Anmeldenummer: 98940183.1
(22) Anmeldetag: 08.07.1998
(51) Int. Cl.: A61K 7/00

(54) **MUND- UND ZAHNPFLEGEMITTEL FÜR EMPFINDLICHE ZÄHNE**
ORAL AND DENTAL HYGIENE PRODUCTS FOR SENSITIVE TEETH
AGENTS DE SOIN A USAGE BUCCAL ET DENTAIRE POUR DENTS SENSIBLES

(30) Priorität: 17.07.1997 DE 19730651
(43) Veröffentlichungstag der Anmeldung: 17.05.2000
(73) Patentinhaber: Henkel Kommanditgesellschaft auf Aktien, 40589 Düsseldorf-Holthausen (DE)
(72) Erfinder: LEINEN, Hans, Theo, D-40229 Düsseldorf (DE); WÜLKNITZ, Peter, D-42799 Leichlingen (DE)
(86) Internationale Anmeldenummer: EP9804245
(87) Internationale Veröffentlichungsnummer: WO99003445

(56) Entgegenhaltungen:
- EP-A- 0 346 957
- WO-A-92/04006
- WO-A-93/24103
- WO-A-96/03109
- WO-A-97/04742
- WO-A-97/28782
- US-A- 3 888 976
- US-A- 4 407 675

## Beschreibung

Die Erfindung betrifft Mund- und Zahnpflegemittel mit einer Wirkstoffkombination zur Verringerung der Sensibilität der Zähne gegen mechanische und thermische Reize.

Manche Menschen leiden unter einer extremen Empfindlichkeit der Zähne, insbesondere im Bereich der Zahnhälse und verspüren ein unangenehmes Gefühl bis hin zum Schmerz, wenn die Zähne z.B. beim Genuß gekühlter Speisen oder Getränke oder bei der Berührung mit der Zahnbürste einem thermischen oder mechanischen Reiz ausgesetzt werden.

Es ist bekannt, daß wasserlösliche Salze des Kaliums und Strontiums diese Unempfindlichkeit in gewissem Umfang verringern können. So wird z.B. in DE 24 19 384 ein Zusatz von Kaliumnitrat, in US 3,122,483 der Zusatz von Strontiumchlorid zu Zahnpasten empfohlen.

Die Verwendung von Nelkenknospenöl (Nelkenöl, clove oil) und dessen Hauptbestandteil Eugenol, als Aromaöl mit antibakteriellen Eigenschaften in Mund- und Zahnpflegemitteln, ist ebenfalls schon lange bekannt, z.B. aus der deutschen Patentschrift DE 24 45 676 C2.

Aus der US 4,407,675 ist weiterhin ein Zahnfüllmaterial bekannt, das eine Mischung aus Zinkoxid und Eugenol zur Erreichung einer zementähnlichen Konsistenz, sowie einen Anteil von Kaliumnitrat zur Vorbeugung der Zahnfleisch-Degeneration, enthält.

Die US 3,888,976 beschreibt eine Tablette, die in Wasser aufgelöst zur Mund- und Zahnpflege verwendet wird und Strontiumionen oder Zinkionen als adstringierendedesensibilisierende Komponenten enthält.

Ferner werden in der WO 92/04006 Verfahren zur Herstellung desensibilisierender Kompositionen, enthaltend Calcium- oder Strontiumionen und in der EP 0 346 957 Mittel zur Behandlung sensitiver Zähne, die Kalium- oder Strontiumionen enthalten, offenbart.

Es wurde nun aber gefunden, daß ein Gehalt an Eugenol oder an Nelkenknospenöl die desensibilisierende Wirkung von Kalium- oder Strontiumsalzen auf überempfindliche Zähne noch erheblich steigert. Insbesondere wurde ein rascheres Eintreten der Wirkung beobachtet. Eine solche, im Zusammenwirken mit anderen desensibilisierend wirkenden Stoffen potenzierende Wirkung des Eugenols war bisher unbekannt.

Gegenstand der Erfindung sind daher Mund- und Zahnpflegemittel in Form von wäßrigen, wäßrig-alkoholischen oder auch wasserfreien Zubereitungen, die zur Desensibilisierung empfindlicher Zähne eine Wirkstoffkombination aus einem wasserlöslichen Kalium- oder Strontiumsalz und Eugenol oder Nelkenblütenöl enthalten.

Die erfindungsgemäßen Mund- und Zahnpflegemittel können in Form von Zahnpasten, flüssigen Zahncremes, Zahnpulvern, Mundwässern oder gegebenenfalls auch als Kaumassen, z.B. Kaugummi vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.

In den gebrauchsfertigen Zubereitungen sind vorzugsweise wenigstens 0,5 Gew.-% Kalium- oder Strontium-Ionen in Form eines gelösten Salzes und wenigstens 0,01 Gew.-% Eugenol entweder in reiner Form oder in Form des Nelkenknospenöls enthalten. Nelkenknospenöl enthält in der Regel 70 - 90 Gew.-% Eugenol neben 10 - 15 Gew.-% Eugenolacetat (Geruchskomponente) sowie kleinere Anteile an Alkoholen, Methylestern, Ketonen und Sesquiterpenen.

Geeignete wasserlösliche Salze des Kaliums sind z.B. Kaliumchlorid, Kaliumsulfat, Kaliumbicarbonat, Kaliumcitrat, Kaliumacetat, Kaliumlactat und Kaliumnitrat. Bevorzugt eignet sich Kaliumnitrat.

Geeignete wasserlösliche Salze des Strontiums sind Strontiumchlorid, Strontiumnitrat, Strontiumcitrat, Strontiumacetat und Strontiumlactat.

Die Salze des Kaliums oder Strontiums sind bevorzugt in einer Menge von 0,5 - 10 Gew.-% (berechnet als K- oder Sr-Ion) enthalten.

Das Eugenol kann auch mit anderen Aromaölen gemischt in den Mund- und Zahnpflegemitteln enthalten sein. Bevorzugt ist es in Form des Nelkenknospenöls in einer Menge von 0,01 bis 1 Gew.-% (berechnet als Eugenol) in den Zusammensetzungen enthalten.

Erfindungsgemäße Zahnpasten oder flüssige Zahncremes enthalten ein Poliermittel, üblicherweise in einer Menge von 5 bis 50 Gew.-%, sowie ein Feuchthaltemittel, gewöhnlich in einer Menge von 10 - 60 Gew.-%.

Als Poliermittel eignen sich alle für Zahnpasten bekannten Reibkörper wie z.B. Kieselsäuren, Aluminiumhydroxid, Aluminiumoxid, Calciumpyrophosphat, Kreide, Dicalciumphosphat-dihydrat, Natrium-aluminiumsilikate z.B. Zeolith A, organische Polymere z.B. Polymethacrylat oder Gemische dieser Reibkörper. Als besonders geeignet hat sich der Zusatz eines Poliermittels erwiesen, das selbst eine restaurierende Wirkung auf Läsionen und offene Dentalkanälchen hat, das Dicaiciumphosphat-dihydrat. Dicalciumphosphat-dihydrat (CaHPO₄ · 2H₂O) kommt in der Natur als Brushit vor und ist im Handel in geeigneten Korngrößen von 1 bis 50 µm erhältlich.

Eine besonders bevorzugte Ausführung der Erfindung sind daher Mund- und Zahnpflegemittel in Form einer Zahnpaste oder Zahncreme mit 5 - 50 Gew.-% eines Poliermittels und 10 - 60 Gew.-% eines Feuchthaltemittels, die als Poliermittelkomponente wenigstens 1 Gew.-% Dicalciumphosphat-dihydrat enthalten. Eine besonders wirksame Poliermittelkombination besteht aus 10 - 20 Gew.-% Kieselsäure und 1 - 10 Gew.-% Dicalciumphosphat-dihydrat (wobei die %-Angaben sich jeweils auf die fertige Zahnpaste beziehen).

Als Kieselsäurepoliermittel eignen sich alle als Putzkörper bekannten Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalt von 15 bis 35 Gew.-%, so werden die sogenannten Hydrogelkieselsäuren erhalten, wie sie z.B. aus US 4,153,680 bekannt sind. Durch Trocknung auf Wassergehalte unterhalb 15 Gew.-% erfolgt eine irreversible Schrumpfung der vorher lockeren Struktur des Hydrogels zur dichten Struktur des sog. Xerogels. Solche Xerogelkieselsäuren sind z.B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet ist eine gemäß DE-OS 31 14 493 hergestellte Fällungskieselsäure mit einer BET-Oberfläche von 15 - 110 m²/g, einer Partikelgröße von 0,5 - 20 µm, wobei wenigstens 80 Gew.-% der Primärpartikel unter 5 m liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste.

Bevorzugt geeignete Fällungskieselsäuren weisen außerdem gerundete Ecken und Kanten auf und sind unter der Handelsbezeichnung Sident®12 DS (DEGUSSA) erhältlich.

Um einen genügend hohen Anteil an Kieselsäuren mit einer mittleren Teilchengröße von weniger als 5 µm und besonders um einen Anteil von wenigstens 3 Gew.-%, bezogen auf die gesamte Zahnpaste, an Kieselsäure mit einer Primärpartikelgröße von 1 - 3 µm zu erhalten, setzt man bevorzugt neben der genannten Fällungskieselsäure noch eine feinteiligere Type mit einer BET-Oberfläche von 150 - 250 ein. Eine geeignete Kieselsäuretype ist z.B. Sipernat®22LS (DEGUSSA), die in einer Menge von 1 - 5 Gew.-% der Zahnpaste eingesetzt wird.

Es hat sich als besonders vorteilhaft für die Ausbildung einer glatten Zahnoberfläche erwiesen, wenn die erfindungsgemäße Zahnpaste geringe Mengen von frisch gefällter, d.h. in situ bei der Pastenherstellung erzeugter Kieselsäure enthält. Dies wird z.B. dadurch erreicht, daß man bei der Herstellung der Zahnpaste durch z.B. Citronensäure einen pH-Wert von 3 - 5 einhält und dann durch Zusatz geringer Mengen einer wäßrigen Natriumsilikatlösung den pH-Wert auf 7 - 7,5 anhebt. Die auf diese Weise in situ gebildete Kieselsäure ist äußerst feinteilig und macht weniger als 0,1 Gew.-% der Zahnpaste aus.

Als weitere Poliermittelkomponente kann auch z.B. Aluminiumoxid in Form von schwach kalzinierter Tonerde mit einem Gehalt an γ- und α-Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Es wurde weiterhin beobachtet, daß Magnesiumionen einen günstigen Einfluß auf das kontrollierte Kristallwachstum des in den Zahnläsionen wachsenden Hydroxylapatit haben, die beschleunigte Härtung des Zahnemails fördern, und daher einen weiteren Beitrag zur Lösung der erfindungsgemäßen Aufgabe leisten. Ein weiterer Gegenstand der Erfindung ist daher eine erfindungsgemäße Zahnpaste, die ein wasserlösliches Magnesiumsalz in einer Menge entsprechend 0,1 - 0,5 Gew.-% Mg enthält. Als Magnesiumsalze eignen sich alle Salze, die in Wasser bei 20°C wenigstens zu 0,5 Gew.-% (berechnet als Mg) löslich sind, z.B. MgSO₄, MgCl₂ und Magnesium-monofluorophosphat (MgPO₃F).

Schließlich wurde festgestellt, daß auch Fluorophosphat-Ionen einen günstigen Einfluß auf das kontrollierte Kristallwachstum des Hydroxylapatits haben. Bevorzugt sind daher erfindungsgemäße Zahnpasten, die als Fluorverbindung ein Monofluorophosphat in einer Menge entsprechend 0,2 - 2,0 Gew.-% (PO₃F⁻) enthalten. Es eignen sich hierfür z.B. die Alkalisalze, z.B. das handelsübliche Natrium-monofluorophosphat. Der bevorzugte Gehalt an Magnesiumionen und an Fluorophosphationen läßt sich besonders vorteilhaft dadurch erreichen, daß als Fluorverbindung Magnesiummonofluorophosphat enthalten ist.

Als Träger für die erfindungsgemäßen Zahnpasten, der die Einstellung einer geeigneten Konsistenz für die Dosierung aus Tuben, Spendebehältern oder flexiblen Flaschen auf der Grundlage der erfindungsgemäßen Poliermittelkombination ermöglicht, eignet sich eine Kombination aus Feuchthaltemitteln, Bindemitteln und Wasser. Als Feuchthaltemittel können z.B. Glycerin, Sorbit, Xylit, Propylenglycole, Polyethylenglycole, insbesondere solche mit mittleren Molekulargewichten von 200 - 800 eingesetzt werden. Als Konsistenzregler (bzw. Bindemittel) dienen z.B. natürliche und/oder synthetische wasserlösliche Polymere wie Alginate, Carragheenate, Traganth, Stärke und Stärkeether, Celluloseether wie z.B. Carboxymethylcellulose (Na-Salz), Hydroxyethylcellulose, Methylhydroxypropylcellulose, Guar, Akaziengum, Agar-Agar, Xanthan-Gum, Succinoglycan-Gum, Johannisbrotmehl, Pectine, wasserlösliche Carboxyvinylpolymere (z.B. Carbopol®-Typen), Polyvinylalkohol, Polyvinylpyrrolidon, Polyethylenglycole, insbesondere solche mit Molekulargewichten von 1 500 - 1 000 000.

Weitere Stoffe, die sich zur Viskositätskontrolle eignen, sind z.B. Schichtsilikate wie z.B. Montmorillonit-Tone, kolloidale Verdickungskieselsäuren wie z.B. Aerogel-Kieselsäuren, pyrogene Kieselsäuren oder feinstvermahlene Fällungskieselsäuren.

Die Zahnpasten können in ihrer Viskosität auch so niedrig eingestellt sein, daß sie sich als "flüssige Zahnreinigungsmittel" mit einer Viskosität von 2 000 - 10 000 m·Pa·s (25°C) aus einer flexiblen Kunststoffflasche auf die Zahnbürste dosieren lassen, dort zwischen die Borsten eindringen, aber nicht von der Zahnbürste abtropfen. Für diesen Zweck eignet sich als Bindemittel bevorzugt eine Kombination aus 0,1 - 1 Gew.-% Xanthan-Gum und 0,01 - 5 Gew.-% eines viskositätsstabilisierenden Zusatzes aus der Gruppe
- der kationischen, zwitterionischen oder ampholytischen stickstoffhaltigen Tenside
- der hydroxypropylsubstituierten Hydrocolloide oder
- der Polyethylenglycol/Polypropylenglycol-Copolymere mit einem mittleren Molgewicht von 1000 bis 5000
oder einer Kombination der genannten Verbindungen.

Auch oberflächenaktive Substanzen sind in den erfindungsgemäßen Zahnpasten zur Unterstützung der Reinigungswirkung und gewünschtenfalls auch zur Entwicklung von Schaum beim Zähnebürsten sowie zur Stabilisierung der Polierkörperdispersion im Träger in einer Menge von 0,1 - 5 Gew.-% enthalten.

Geeignete Tenside sind z.B. lineare Natriumalkylsulfate mit 12 - 18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12 - 16 C-Atomen in der linearen Alkylgruppe und 2 - 6 Glycolethergruppen im Molekül, von linearem Alkan(C₁₂-C₁₈)-sulfonat, von Sulfobernsteinsäuremonoalkyl(C₁₂-C₁₈)-estern, von sulfatierten Fettsäuremonoglyceriden, sulfatierten Fettsäurealkanolamiden, Sulfoessigsäurealkyl(C₁₂-C₁₆)-estern, Acylsarcosinen, Acyltauriden und Acylisethionaten mit jeweils 8 - 18 C-Atomen in der Acylgruppe. Auch zwitterionische, ampholytische und nichtionische Tenside sind geeignet, z.B. Oxethylate von Fettsäuremono- und -diglyceriden, von Fettsäure-Sorbitanestern und Alkyl(oligo)-Glucoside.

Weitere übliche Zahnpastenzusätze sind
- Konservierungsmittel und antimikrobielle Stoffe, wie z.B. p-Hydroxybenzoesäuremethyl-, -ethyl- oder propylester, Natriumsorbat, Natriumbenzoat, Bromchlorophen, Triclosan, Phenyl-salicylsäureester, Biguanide z.B. Chlorhexidin, Thymol usw.
- Süßungsmittel wie z.B. Saccharin-Natrium, Natrium-Cyclamat, Sucrose, Lactose, Maltose, Fructose,
- Aromen wie z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen,
- Pigmente wie z.B. Titandioxid
- Farbstoffe
- Puffersubstanzen wie z.B. primäre, sekundäre oder tertiäre Alkaliphosphate oder Citronensäure/Natriumcitrat,
- wundheilende und entzündungshemmende Stoffe wie z.B. Allantoin, Harnstoff sowie Azulen, Kamillewirkstoffe, Acetylsalicylsäurederivate.

In Mundwässern besteht der Träger im wesentlichen aus Wasser, Ethanol, etherischen Ölen, Emulgatoren und Lösungsvermittlern für das Eugenol und die übrigen Aromakomponenten, Geschmackskorrigentien (z.B. Süßstoff) sowie gegebenenfalls adstringierenden oder belebenden Drogenauszügen und gegebenenfalls Farbstoffen. Als weitere Wirkstoffe können neben dem desensibilisierenden Kalium- oder Strontiumsalz und dem Eugenol noch antimikrobielle Stoffe wie z.B. Chlorhexidin oder Triclosan enthalten sein.

Die folgenden Beispiele sollen den Erfindungsgegenstand näher erläutern:

### Beispiele

Es wurden die folgenden Mund- und Zahnpflegemittel hergestellt:
**1. Zahnpasten**

| | **1** | **2** | **3** | **4** |
|---|---|---|---|---|
| Fällungskieselsäure : Sident®12 DS | 10,0 | 12 | 15 | 18 |
| Fällungskieselsäure : Sipernat®22 LS | 3,0 | 3,0 | - | - |
| Poliertonerde P10 feinst (1) | - | - | - | 1,0 |
| Dicalciumphosphat-dihydrat | 4,0 | 4,0 | - | - |
| MgSO₄ ^{.}7H₂O | 1,7 | 1,4 | - | - |
| Na-Monofluorophosphat Na₂PO₃F | 1,2 | 0,8 | 0,5 | 0,6 |
| KNO₃ (wasserfrei) | 5,0 | 4,0 | 4,5 | 3 |
| Glycerin (86 % DAB) | 21,0 | 18,0 | 10 | 17,5 |
| Sorbit (70 % DAB) | 20,0 | 14,0 | 5 | 17,5 |
| Polyethylenglycol (MG : 400) | 2,0 | 2,0 | 1,0 | 1,0 |
| Verdickungskieselsäure (FK 320 DS) | 1,0 | 1,0 | 5,0 | 0,8 |
| Xanthan-Gum (Keltrol®F) | 0,6 | 0,6 | 0,6 | 0,5 |
| Titandioxid | 1,0 | 1,0 | - | - |
| Na-Laurylsulfat | 1,5 | 1,5 | - | 2,0 |
| Tego Betain BL 215 (2) | 0,6 | 0,6 | - | - |
| Trinatriumcitrat | 0,2 | 0,2 | - | - |
| Saccharin-Na | 0,2 | 0,2 | 0,1 | 0,2 |
| Nelkenknospenöl | 0,05 | 0,1 | 0,07 | 0,1 |
| Aroma | 1,0 | 0,8 | 0,1 | 1,0 |
| Cremophor RH60 (3) | - | - | 0,2 | - |
| Wasser | ad 100 | ad 100 | ad 100 | ad 100 |

**2. Flüssige Zahncreme**

| | **5** |
|---|---|
| Fällungskieselsäure (Sident 12 DS) | 12,0 |
| NaF | 0,25 |
| KNO₃ | 5,0 |
| Na₂HPO₄ | 0,2 |
| Xanthan-Gum (Keltrol F) | 0,2 |
| Azacycloheptan-2,2-diphosphonat (Na-Salz) | 1,0 |
| Saccharin-Na | 0,2 |
| Ethanol | 5,0 |
| Glycerin | 28,0 |
| Sorbit | 22,0 |
| Polyethylenglycol (MG : 400) | 3,0 |
| PEG 30 - Glycerinmonostearat | 1,0 |
| Tego Betain BL 215 (2) | 0,8 |
| Aromaöl | 1,0 |
| Nelkenknospenöl | 0,1 |

**3. Mundwasser (gebrauchsfertig)**

| | **6** |
|---|---|
| Ethanol | 5,0 |
| Chlorhexidin-gluconat | 0,03 |
| Plantaren 2000 (4) | 0,05 |
| Na₂PO₃F | 0,25 |
| KNO₃ | 4,0 |
| Saccharin-Na | 0,05 |
| Sorbit | 3,0 |
| Cremophor RH 60 (3) | 0,1 |
| Aromaöl (Pfefferminzöl) | 0,1 |
| Nelkenknospenöl | 0,05 |
| Farbstoff (blau, C.J. 42090) | 0,1 |
| Wasser | ad 100 |

Es wurden die folgende Handelsprodukte verwendet:
- (1) Poliertonerde P10 feinst :: Schwach calcinierte Tonerde (ca. 20 Gew.-% Gamma-Aluminiumoxid ca. 80 Gew.-% Alpha-Aluminiumoxid Primärkristallgröße 0,5 - 1,5 um)
- (2) Tego® Betain BL 215 :: 30 %ige Lösung von Cocoamidopropyl-Betain in Wasser
- (3) Cremophor®RH 60 :: Hydr. Rizinusöl + 60 Mol EO
- (4) Plantaren 2000 :: Alkyl-(C₈-C₁₆)-oligo-(1,4)-glucosid (50 %ige Lösung in Wasser)

### Klinischer Wirkungsnachweis

In den klinischen Vergleichen wurden eingesetzt:
- E =: Beispiel 1
- V1 =: Beispiel 1 ohne Nelkenknospenöl (Ersatz durch Wasser)
- VM =: Sensitive-Zahnpaste (Marktprodukt)

### Testmethodik:

Es wurden drei Testgruppen mit je 50 Testpersonen gebildet. Dabei wurde die Zahnsensibilität zu Beginn der Studie und nach Abschluß der 8-wöchigen Testzeit durch einen Zahnarzt festgestellt. Parallel wurden individuelle Beurteilungen anhand eines Fragebogens ermittelt.
Die zahnärztliche Prüfung erfolgte mit einem Luftstrahl, der auf den Zahnhals gerichtet wurde.
Es handelte sich um eine Doppelblind-Studie. Vor Testbeginn wurden die Zähne 2 x täglich mit einer neutralen Zahnpaste ohne desensibilisierende Zusätze gereinigt.
Während der Testphase wurde von jeder der drei Gruppen eine der drei Test-Zahncremes in üblicherweise 2 x täglich angewendet.
Als Testpersonen wurden nur solche Personen eingesetzt, die bei der ersten Prüfung am Testbeginn eine Zahnsensibilität aufwiesen, die der Benotung 2 oder höher entspricht.

Die Benotung erfolgte auf einer fünfstufigen Skala von
0 = völlig unempfindlich über
1 = sehr geringe Empfindlichkeit
2 = geringe Empfindlichkeit
3 = mittlere Empfindlichkeit
4 = hohe Empfindlichkeit
5 = sehr hohe Empfindlichkeit

Ein statistischer Gruppen-Vergleich wurde nach dem Log Rank-Test (nach Peto-Wilcoxon) durchgeführt.

Die folgende Tabelle I zeigt die Ergebnisse der Sensibilitäts-Prüfung für die Personen, die den Test bis zu Ende durchgeführt haben.

**Tabelle I**

| | **Gruppe E** | | **Gruppe V1** | | **Gruppe VM** | |
|---|---|---|---|---|---|---|
| Beurteilung | Start | Ende | Start | Ende | Start | Ende |
| 0 | - | 1 | - | 1 | - | 3 |
| 1 | 0 | 13 | 2 | 8 | 2 | 6 |
| 2 | 14 | 16 | 7 | 8 | 4 | 5 |
| 3 | 21 | 9 | 13 | 16 | 8 | 8 |
| 4 | 6 | 4 | 12 | 5 | 14 | 13 |
| 5 | 3 | 1 | 9 | 5 | 17 | 10 |
| Summe | 44 | 44 | 43 | 43 | 45 | 45 |

Die folgenden Tabellen geben für jede der Prüfgruppen Aufschluß über die Zahl der Testpersonen, die von einer bestimmten Beurteilung (1 - 5) vor Beginn des Tests (senkrecht) zu einer bestimmten Beurteilung (1 - 5) am Testende (waagrecht) gelangt sind.

Man erkennt, daß in der Gruppe, die das erfindungsgemäße Produkt geprüft hat (Gruppe E) nach dem Test nur 5 Personen die Beurteilung 4 oder 5 und 30 Personen die Beurteilung 0 bis 2 abgaben.

**Tabelle II**

| **Produkt: E** | | | | | | | |
|---|---|---|---|---|---|---|---|
| vorher/nachher | 0 | 1 | 2 | 3 | 4 | 5 | Summe |
| 0 | | | | | | | |
| 1 | | | | | | | |
| 2 | | 6 | 7 | 1 | | | 14 |
| 3 | 1 | 4 | 8 | 7 | 1 | | 21 |
| 4 | | 1 | 1 | 1 | 2 | 1 | 6 |
| 5 | | 2 | | | 1 | | 3 |
| Summe | 1 | 13 | 16 | 9 | 4 | 1 | 44 |

**Tabelle III**

| **Produkt: V1** | | | | | | | |
|---|---|---|---|---|---|---|---|
| vorher/nachher | 0 | 1 | 2 | 3 | 4 | 5 | Summe |
| 0 | | | | | | | |
| 1 | | | 1 | | | 1 | 2 |
| 2 | | 2 | 2 | 2 | 1 | | 7 |
| 3 | | 5 | 2 | 3 | 1 | 2 | 13 |
| 4 | 1 | 1 | 1 | 7 | 2 | | 12 |
| 5 | | | 2 | 4 | 1 | 2 | 9 |
| Summe | 1 | 8 | 8 | 16 | 5 | 5 | 43 |

**Tabelle IV**

| **Produkt : VM** | | | | | | | |
|---|---|---|---|---|---|---|---|
| vorher/nachher | 0 | 1 | 2 | 3 | 4 | 5 | Summe |
| 0 | | | | | | | |
| 1 | 1 | 1 | | | | | 2 |
| 2 | | 2 | 2 | | | | 4 |
| 3 | 1 | 2 | 1 | 3 | 1 | | 8 |
| 4 | 1 | 1 | 2 | 5 | 4 | 1 | 14 |
| 5 | | | | | 8 | 9 | 17 |
| Summe | 3 | 6 | 5 | 8 | 13 | 10 | 45 |

## Patentansprüche

1. Mund- und Zahnpflegemittel in Form einer Zahnpaste oder flüssigen Zahncreme mit 5-50 Gew.-% eines Poliermittels und 10-60 Gew.-% eines Feuchthaltemittels, das zur Desensibilisierung empfindlicher Zähne eine Wirkstoffkombination aus einem wasserlöslichen Kalium- oder Strontiumsalz und Eugenol oder Nelkenknospenöl enthält.

2. Mund- und Zahnpflegemittel nach Anspruch 1, **dadurch gekennzeichnet, daß** die gebrauchsfertige Zubereitung wenigstens 0,5 Gew.-% Kalium- oder Strontiumionen und wenigstens 0,01 Gew.-% Eugenol, bevorzugt in Form des Nelkenknospenöls, enthält.

3. Mund- und Zahnpflegemittel nach einem der Ansprüche 1 oder 2 in Form einer Zahnpaste, **dadurch gekennzeichnet, daß** als Poliermittelkomponente wenigstens 1 Gew.-% Dicalciumphosphat-dihydrat enthalten sind.

4. Mund- und Zahnpflegemittel nach Anspruch 3, **dadurch gekennzeichnet, daß** zusätzlich ein wasserlösliches Magnesiumsalz in einer Menge von 0,1 - 0,5 Gew.-% Mg enthalten ist.

5. Mund- und Zahnpflegemittel nach einem der Ansprüche 1 bis 4, **dadurch gekennzeichnet, daß** eine Fluorverbindung, bevorzugt ein Monofluorphosphat, in einer Menge entsprechend 0,2 - 2,0 Gew.-% (PO₃F⁻) enthalten ist.

## Claims

1. Oral and dental care composition in the form of a toothpaste or liquid tooth cream containing 5 to 50% by weight of a polishing material and 10 to 60% by weight of a humectant, **characterized in that** the composition contains a combination of a water-soluble potassium or strontium salt and eugenol or clove oil as active ingredients for desensitizing sensitive teeth.

2. Oral and dental care composition as claimed in claim 1, **characterized in that** the ready-to-use preparation contains at least 0.5% by weight of potassium or strontium ions and at least 0.01% by weight of eugenol, preferably in the form of clove oil.

3. Oral and dental care composition as claimed in claim 1 or 2 in the form of a toothpaste, **characterized in that** at least 1% by weight of dicalcium phosphate dihydrate is present as a component of the polishing material.

4. Oral and dental care composition as claimed in claim 3, **characterized in that** a water-soluble magnesium salt is additionally present in a quantity of 0.1 to 0.5% by weight Mg.

5. Oral and dental care composition as claimed in any of claims 1 to 4, **characterized in that** a fluorine compound, preferably a monofluorophosphate, is present in a quantity corresponding to 0.2 to 2.0% by weight (PO₃F⁻).

## Revendications

1. Agent de soin buccal et dentaire sous la forme d'une pâte dentifrice ou d'une crème dentifrice liquide avec 5 à 50 % en poids d'un agent de polissage et de 10 à 60 % en poids d'un agent de maintien de l'humidité, qui contient, en vue de désensibiliser les dents sensibles, une combinaison de substances actives, constituée d'un sel de potassium ou de strontium soluble dans l'eau, et d'eugénol ou d'essence de bourgeons de clous de girofle.

2. Agent de soin buccal et dentaire selon la revendication 1,
**caractérisé en ce que**
la préparation prête à l'emploi contient au moins 0,5 % en poids d'ions potassium ou strontium et au moins 0,01 % en poids d'eugénol, de préférence sous forme d'huile de bourgeons de clous de girofle,

3. Agent de soin buccal et dentaire selon l'une des revendications 1 ou 2 sous la forme d'une pâte dentifrice,
**caractérisé en ce qu'**
on y trouve comme composant de polissage au moins 1 % en poids de phosphate dicalcique dihydraté.

4. Agent de soin buccal et dentaire selon la revendication 3,
**caractérisé en ce qu'**
on y trouve en outre un sel de magnésium soluble dans l'eau en une quantité de 0,1 à 0,5 % en poids de Mg.

5. Agent de soin buccal et dentaire selon l'une des revendications 1 à 4,
**caractérisé en ce qu'**
on y trouve un composé de fluor, de préférence un monofluorophosphate, en une quantité allant de 0,2 à 2,0 % en poids (PO₃F-).
